Europäisches Patentamt

European Patent Office

Office européen des brevets

(19)

(11) **EP 1 424 055 A1**

(12) **EUROPÄISCHE PATENTANMELDUNG**

(43) Veröffentlichungstag:
    **02.06.2004  Patentblatt 2004/23**

(51) Int Cl.⁷: **A61G 9/00**, A61B 10/00,
    B01L 3/00

(21) Anmeldenummer: 02026250.7

(22) Anmeldetag: **26.11.2002**

(84) Benannte Vertragsstaaten:
    **AT BE BG CH CY CZ DE DK EE ES FI FR GB GR
    IE IT LI LU MC NL PT SE SK TR**
    Benannte Erstreckungsstaaten:
    **AL LT LV MK RO SI**

(71) Anmelder: **Kunststofftechnik Schedel GmbH
    D-08223 Falkenstein / Vogtland (DE)**

(72) Erfinder: **Weber, Udo
    08223 Falkenstein (DE)**

(74) Vertreter: **Zech, Stefan Markus Dipl.-Phys.
    Patentanwälte
    Meissner, Bolte & Partner
    Bankgasse 3
    90402 Nürnberg (DE)**

(54) **Zur Aufnahme menschlicher Exkremente ausgebildetes, relativ zum Bett eines pflegebedürftigen Patienten positionierbares Steckbecken**

(57)    Bei der Erfindung handelt es sich um ein zur Aufnahme menschlicher Exkremente ausgebildetes, relativ zum Bett eines pflegebedürftigen Patienten positionierbares Steckbecken mit einem durch eine umlaufende Brille (13) teilweise abgedeckten Aufnahmeraum (14). Weiterhin ist eine Montagestelle (16) zur lösbaren Befestigung des Steckbeckens (12) an einem vorzugsweise verfahrbaren Montagearm (27) vorgesehen sowie ein Ablauf (17) zum Anschluss an eine Absaugeinrichtung (18) und ein Zulauf (19) zum Anschluss an eine Spüleinrichtung (20).

FIG 2

**Beschreibung**

Die Erfindung betrifft ein zur Aufnahme menschlicher Exkremente ausgebildetes, relativ zum Bett eines pflegebedürftigen Patienten positionierbares Steckbecken mit einem durch eine umlaufende Brille teilweise abgedeckten Aufnahmeraum.

Steckbecken sind bereits aus dem Stand der Technik bekannt. Sie umfassen meist einen in einem Behältnis ausgebildeten Aufnahmeraum, wobei der Aufnahmeraum des Behältnisses durch einen Deckel abgedeckt sein kann. Derartige Behältnisse, die beispielsweise aus Edelstahl gebildet sein können, werden vom Klinik- oder Pflegepersonal bereitgestellt und müssen dann entweder unmittelbar nach Benutzung durch einen Patienten oder, was gerade bei geruchsintensiveren Exkrementen lästig sein kann, bei Gelegenheit vom Klinik- oder Pflegepersonal abgeholt, der Inhalt entsorgt und das Behältnis gereinigt werden. Für den Patienten ist diese Prozedur sehr unangenehm, da er einerseits dafür sorgen muss, das zur Entleerung von Exkrementen ein frisches Steckbecken bereitsteht; andererseits muss er das Klinik- oder Pflegepersonal mit der Entsorgung von darin aufgenommenen Exkrementen behelligen.

Die Aufgabe der vorliegenden Erfindung besteht darin, ein Exkremente eines pflegebedürftigen Patienten aufnehmendes Steckbecken dahingehend weiterzubilden, dass dessen Verwendung für den pflegebedürftigen Patienten nicht mit den oben angegebenen Nachteilen behaftet ist, und das gleichzeitig die Arbeit des Klinik- oder Pflegepersonals erleichtert.

Diese Aufgabe wird mit einem Steckbecken nach den Merkmalen des Anspruches 1 gelöst.

Vorteilhafte Weiterbildungen sind in den Unteransprüchen angegeben.

Nach einem Kerngedanken der vorliegenden Erfindung umfasst das Steckbecken eine Montagestelle zur lösbaren Befestigung des Steckbeckens an einem vorzugsweise verfahrbaren Montagearm, einen Ablauf zum Anschluss an eine Absaugeinrichtung und einen Zulauf zum Anschluss an eine Spüleinrichtung.

Das erfindungsgemäße Steckbecken zeichnet sich also dadurch aus, dass es einerseits wie die nach dem Stand der Technik bekannten Steckbecken bedarfsweise an oder in das Bett des pflegebedürftigen Patienten positioniert werden kann, gleichzeitig aber auch, wenn es nicht benötigt wird, wieder aus dem Bett entfernt werden kann. Gleichzeitig kann das an eine Absaugeinrichtung bzw. an eine Spüleinrichtung angeschlossene Steckbecken automatisch entleert bzw. gespült werden. In einer vorteilhaften Ausgestaltung sind Zulauf und Ablauf des Steckbeckens um 20 bis 160°, vorzugsweise etwa 70 bis 110°, insbesondere etwa 90° zueinander versetzt angeordnet. Diese Ausbildung bietet Vorteile in der Handhabung, insbesondere bei Absaugung und Spülung.

Da das Steckbecken nach der Erfindung insbesondere auch zum Absetzen von Stuhl und/oder Urin geeignet sein soll, erscheint es vorteilhaft, die Schüssel bzw. die Brille an einer Seite zu einer Schutzwand hochzuziehen, so dass an einer dem Körper des Patienten zugewandten Seite das Steckbecken gut zugänglich, an einer dem Körper des Patienten abgewandten Seite dagegen mit einer Schutzwand versehen ist, um die Exkremente sicher aufzunehmen und in den Aufnahmeraum der Schüssel zu führen.

Nach einem weiter bevorzugten Aspekt der vorliegenden Erfindung ist die umlaufende Brille lösbar auf der Schüssel befestigt. Diese Ausgestaltung empfiehlt sich, um neben der unmittelbar nach Benutzung vorgesehenen Spülung von Zeit zu Zeit auch eine gründliche Reinigung der Schüssel und/oder der Brille vornehmen zu können. Eine lösbar befestigbare Ausgestaltung der Brille auf der Schüssel kann sich auch empfehlen, sofern Brille und Schüssel entsprechend symmetrisch ausgestaltet sind, um die Brille auf der Schüssel um 180° versetzt anzuordnen. Es kann so ein Steckbecken mit rechter Ausrichtung, d.h. Anordnung des Ablaufes rechts von der Schutzwand, bzw. mit einer linken Ausrichtung, d.h. Anordnung des Ablaufes links von der Schutzwand, gewechselt werden.

Nach einem weiteren Aspekt der vorliegenden Erfindung ist die Schutzwand oberhalb der umlaufenden Brille angeordnet. Sie kann einstückig mit der Brille oder als separates Bauteil ausgebildet sein. Auch kann sie die Brille von der Schüssel ausgehend überragen derart, dass die Brille in einer abnehmbaren Ausgestaltung separat abgenommen werden kann. Alternativ kann bei Vorsehen der Schutzwand auf der Brille bei lösbarer Ausgestaltung der Brille auch die Schutzwand mit abgenommen werden.

Nach einem weiteren Aspekt der vorliegenden Erfindung steht der Zulauf mit einer Öffnung in der Schutzwand in Fluidverbindung. Das heißt, über den Zulauf eingebrachtes Spülfluid kann direkt aus der Schutzwand austreten. Alternativ oder zusätzlich kann der Zulauf aber auch mit einer, vorzugsweise einer Mehrzahl düsenartiger Auslässe in Fluidverbindung stehen, wobei die düsenartigen Auslässe an geeigneter Position, beispielsweise im Bereich unterhalb der Brille, an der Brille selbst oder an der Schüssel ausgebildet sein können. Alternativ oder zusätzlich können die düsenartigen Auslässe auch im Bereich der hochgezogenen Schutzwand vorgesehen sein.

Nach einem weiteren Aspekt der vorliegenden Erfindung kann in der Schüssel ein Absauganschluss für einen Geruchsabzug vorgesehen sein. Dieser Absauganschluss kann als weiterer separater Anschluss vorgesehen sein oder in dem bereits erwähnten Zulauf oder Ablauf des Steckbeckens integriert sein. Alternativ oder zusätzlich zu einem Geruchsabzug kann auch ein geruchsneutralisierender bzw. ein Duftstoff durch entsprechende Einrichtungen eingebracht werden.

Nach einem weiteren, besonders vorteilhaften Aspekt der vorliegenden Erfindung ist ein Trocknungsanschluss vorgesehen, um insbesondere durch einen Luftstrom eine rasche Trocknung der Schüssel des Steckbeckens zu bewirken.

In einer besonders bevorzugten Ausgestaltung weist die Schüssel in ihrer Projektion (in Gebrauchslage von oben) eine ovale oder elliptische Grundform auf mit einer maximalen Ausdehnung in eine erste Vorzugsrichtung und einer minimalen Ausdehnung in eine zweite Vorzugsrichtung, wobei erste Vorzugsrichtung und zweite Vorzugsrichtung zueinander orthogonal liegen.

Nach einem weiteren, besonders bevorzugten Aspekt der vorliegenden Erfindung ist der Ablauf dabei an einer der Schmalseiten der Schüssel angeordnet. Bei dieser Ausgestaltung lässt sich das Steckbecken so verwenden, dass die erste Vorzugsrichtung, in der die Schüssel eine maximale Ausdehnung aufweist, im Wesentlichen parallel zur Längsrichtung des Bettes des Patienten verläuft. Wenn sich nun der Ablauf an einer Schmalseite der Schüssel befindet, kann über eine Rohr- oder eine Schlauchverbindung eine Absaugung der Exkremente quer zur Längserstreckung des Bettes erfolgen.

Bevorzugt wird eine ovale oder elliptische Grundform mit einem Verhältnis von maximaler und minimaler Ausdehnung im Bereich zwischen

$$1{,}1 < A_{max:} : A_{min} < 1{,}3$$

und wenn vorzugsweise $A_{max:} : A_{min}$ ungefähr 1,2 beträgt.

Weiterhin wird eine flachbauende Ausgestaltung der Schüssel bevorzugt dergestalt, dass sie an ihren Schmalseiten eine mittlere Höhe von ungefähr 60 bis 100 mm, vorzugsweise 80 mm aufweist.

Die Schüssel kann bereichsweise, beispielsweise in Gestalt einer Schutzwand an einer Seite hochgezogen sein. Genauso kann sie an einer gegenüberliegenden Seite etwas niedriger ausgestaltet sein. Als Mindesthöhe bzw. Mindesttiefe der Schüssel wird jedoch ein Maß von 50 bis 60 mm bevorzugt. Eine mittlere Höhe bzw. Tiefe ohne Berücksichtigung einer hochgezogenen Schutzwand sollte 60 bis 100 mm, vorzugsweise etwa 80 mm betragen.

Nach einem weiteren bevorzugten Aspekt der vorliegenden Erfindung weist die Innenseite der Schüssel einen im Wesentlichen flachen Bodenabschnitt auf.

Um die Reinigungsfähigkeit zu verbessern, können an der Schüssel ein oder mehrere mechanische Reinigungsgeräte angeordnet sein. Die Reinigungsgeräte können im Bereich der Schutzwand, insbesondere innerhalb der Öffnung in der Schutzwand angeordnet sein, und von dort von einer Ruhestellung in eine Arbeitsposition verfahren werden. Das mindestens eine Reinigungsgerät kann aber auch anderweitig mit der Schüssel des erfindungsgemäßen Steckbeckens zusammenwirken.

Obwohl eine von der Rotationsform abweichende Ausgestaltung der Schüssel des Steckbeckens bevorzugt wird, kann die Schüssel selbstverständlich auch eine vorzugsweise im Wesentlichen rotationssymmetrische Gestalt in ihrer Projektion (in Gebrauchslage von oben) einnehmen.

Nach einem weiteren Aspekt der vorliegenden Erfindung nimmt die Krümmung der Innenfläche der Schüssel des Steckbeckens von dem im Wesentlichen flachen Bodenabschnitt zu einem Rand der Schüssel nach oben hin zu. Diese Zunahme kann entweder kontinuierlich oder in einem oder mehreren diskreten Schritten erfolgen.

In einer konkreten, bevorzugten Ausgestaltung schließt sich an den im Wesentlichen flachen Bodenabschnitt ein gekrümmter Bodenabschnitt mit einem Krümmungsradius im Bereich zwischen 160 mm und 240 mm an. An diesen zweiten Krümmungsbereich kann sich beispielsweise noch ein dritter Krümmungsbereich mit einer nochmals leicht erhöhten Krümmung anschließen.

Schließlich kann das Steckbecken an seiner Unterseite noch eine Auflagefläche zur Auflage des Steckbeckens auf dem Bett des Patienten umfassen.

Die Erfindung betrifft auch eine Vorrichtung zur Absaugung menschlicher Exkremente eines an ein Bett gebundenen Patienten umfassend ein Steckbecken nach einem der Ansprüche 1 bis 14, wobei die Vorrichtung einen Montagearm, an dem das Steckbecken befestigt und relativ zu einem Bett positionierbar ist, eine Absaugeinrichtung, die am Auslauf des Steckbeckens angreift, und eine Spüleinrichtung, die am Zulauf des Steckbeckens anschließbar ist, umfasst.

Durch diese Vorrichtung kann das erfindungsgemäße Steckbecken nach Benutzung gespült bzw. abgesaugt werden. Eine Hinzuziehung des Klinik- oder Pflegepersonals ist nicht erforderlich. Vielmehr steht das Steckbecken nach Absaugung und Spülung für eine erneute Benutzung zur Verfügung. Die Befestigung des Steckbeckens an einem Montagearm gestattet, dieses relativ zu einem Bett eines Patienten in eine gewünschte Position zu bringen bzw. aus dieser Position ohne größeren Aufwand auch wieder zu entfernen.

In einer bevorzugten Ausgestaltung ist der Montagearm verfahrbar, so dass bedarfsweise das Steckbecken an das Bett heran bzw. vom Bett weg verfahren werden kann. In einer besonders vorteilhaften Ausgestaltung ist das am Montagearm befestigte Steckbecken (ggf. unter Einwirkung eines Antriebes) durch. den Patienten selbst relativ zum Bett in der gewünschten Position positionierbar bzw. kann vom Patienten selbst vom Bett weg verfahren werden.

In einer zweckmäßigen Ausgestaltung der erfindungsgemäßen Vorrichtung ist zwischen der Absaugeinrichtung und der Schüssel des Steckbeckens eine Geruchssperre angeordnet. Die Geruchssperre kann beispielsweise in Gestalt einer Rückschlagklappe, eines mit Spülfluid gefüllten U-förmigen Rohres, eines Schiebers o. ä. ausgebildet sein.

Der Montagearm ist vorzugsweise schwenkbar und/oder längenverstellbar, weiter vorzugsweise automatisch einstellbar.

Nach einem weiter bevorzugten Aspekt der vorliegenden Erfindung kann die Vorrichtung noch mit einer auf dem Bett, unterhalb des Patienten positionierbaren Luftkammer nach Art eines Luftkissens bzw. einer Luftmatratze und einer daran angeschlossenen Luftbefüllungseinrichtung, insbesondere einem Kompressor, derart zusammenwirken, dass der Patient im Bereich seines Rückens bzw. der Lendenwirbelsäule angehoben werden kann, um so für den Patienten eine für das Absetzen von Stuhl oder Urin bequemere Lage relativ zur Schüssel des erfindungsgemäßen Steckbeckens zu schaffen.

Nach einem weiteren Aspekt der vorliegenden Erfindung ist in Strömungsrichtung vor oder nach der Absaugeinrichtung eine Analyseeinrichtung zur Analyse bestimmter Parameter der ausgeschiedenen Exkremente, wie pH-Bestimmung des Urins etc. zwischengeschaltet bzw. zwischenschaltbar. Da bei Klinikaufenthalten oder allgemein bei Pflege eines Patienten oftmals gesundheitsrelevante Daten aus den Exkrementen eines Patienten bestimmt werden müssen und dies für das Pflegepersonal aufwendig, für den Patienten meist unangenehm ist, kann bei der erfindungsgemäßen Vorrichtung vorgesehen werden, wenigstens einen Teil der ansonsten üblichen Analysen von Stuhl oder Urin in einer der Schüssel des Steckbeckens nachgeschalteten Analyseeinrichtung vorzunehmen. Diese Analyseeinrichtung kann auch nur bedarfsweise zwischengeschaltet werden. Alternativ kann ein Teil der abgesaugten Exkremente aber auch abgezweigt werden, um direkt in einer Analyseeinrichtung untersucht zu werden. Selbstverständlich ist es auch möglich, Stuhl- oder Urinproben lediglich abzusondern und die Analysen in herkömmlicher Weise in einem separaten Labor vorzunehmen.

Ein besonderer Aspekt der vorliegenden Erfindung besteht darin, dass Montagearm und Steckbecken derart angeordnet und ausgebildet sind, dass eine Positionierung der Schüssel des Steckbeckens von der Seite des Bettes her möglich ist. Eine Positionierung von den Stirnseiten des Bettes her ist dagegen meist ausgeschlossen, da die Stirnseiten oftmals verbaut sind.

Falls der Montagearm mit einem Antrieb versehen ist, kann dieser Antrieb selbstverständlich pneumatisch, hydraulisch oder mechanisch ausgebildet sein. Dieser Antrieb kann auch über ein programmierbare Steuerung angesteuert werden, so dass eine bestimmte Position der Schüssel des Steckbeckens abgespeichert und bei erneuter Benutzung automatisch wieder angesteuert werden kann.

Im Hinblick auf die Analyse von Exkrementen bzw. zur Geruchsbekämpfung kann es auch vorgesehen sein, im Zusammenhang mit dem Absetzen von Exkrementen bestimmte Substanzen in die Schüssel des Steckbeckens beispielsweise aus einem Reagenzglas o. ä. einzuleiten. Das Steckbecken kann daher mit entsprechenden Mitteln zur Einleitung derartiger Substanzen ausgestattet sein.

Es wird bevorzugt, die Schüssel des Steckbeckens mit einer gut reinigungsfähigen Oberfläche zu versehen. Aus dem Stand der Technik sind sogenannte superglatte, abperlende Oberflächen bekannt, die auch hier zur Anwendung gelangen könnten.

Die Schüssel des Steckbeckens kann aus einem beliebigen geeigneten Material, insbesondere aus Kunststoff, Metall, wie beispielsweise Nirosta, Edelstahl, oder aus Porzellan gebildet sein. In der Herstellung kann bei Verwendung von Metall die Schüssel aus Blech, insbesondere als Tiefziehteil gebildet werden.

Die Erfindung wird nachstehend auch hinsichtlich weiterer Merkmale und Vorteile anhand der Beschreibung von Ausführungsbeispielen und unter Bezugnahme auf in die beiliegenden Zeichnungen näher erläutert.

Hierbei zeigen:

FIG 1    eine Ausführungsform der Vorrichtung zur Absaugung menschlicher Exkremente umfassend ein Steckbecken in einer schematischen perspektivischen Ansicht,

FIG 2    eine Ausführungsform eines Steckbeckens nach der Erfindung in perspektivischer Ansicht,

FIG 3    die Ausführungsform nach FIG 2 in einer Schnittansicht,

FIG 4    die bei der Ausführungsform nach FIG 2 und 3 dargestellte Schüssel in einer Schnittansicht orthogonal zu FIG 3;

FIG 5    die in FIG 4 dargestellte Schüssel in einer Ansicht von oben,

FIG 6    eine modifizierte Ausgestaltung einer für ein Steckbecken verwendbaren Brille in einer Ansicht von oben,

FIG 7    eine modifizierte Ausführungsform des Steckbeckens nach der Erfindung in perspektivischer Ansicht,

FIG 8    eine nochmals modifizierte Ausführungsform eines Steckbeckens nach der Erfindung in perspektivischer Ansicht.

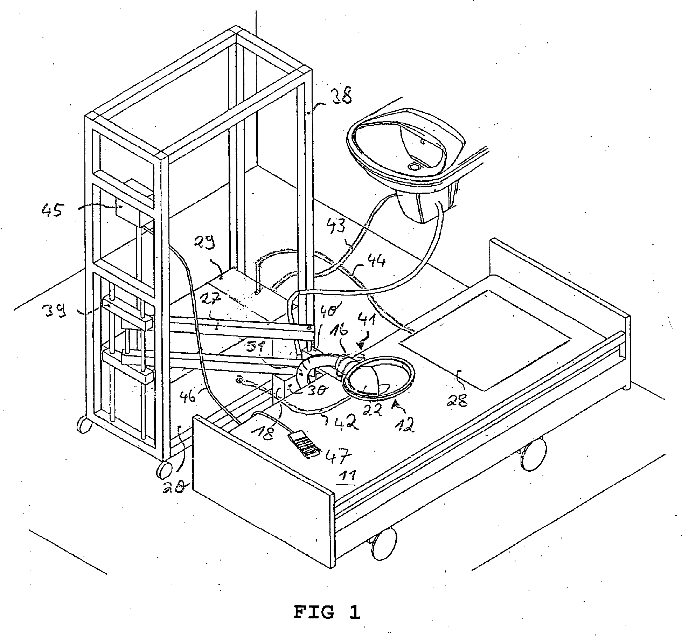

In FIG 1 ist eine Ausführungsform einer erfindungsgemäßen Vorrichtung zur Absaugung menschlicher Exkremente eines an ein Bett 11 gebundenen Patienten in perspektivischer Ansicht schematisch dargestellt. Die Vorrichtung umfasst zunächst ein rahmenartiges Gestell 38, an dem ein schwenkbarer Montagearm 27 um eine vertikale Achse drehbar gelagert ist, Der Montagearm 27 ist ferner über eine Höhenverstelleinrichtung 39 in seiner Höhe einstellbar. Weiter kann der Montagearm 27, was in der vorliegenden Darstellung aber nicht gezeigt ist, auch in seiner wirksamen Länge verfahrbar ausgebildet sein.

An dem Montagearm 27, der bei der vorliegenden Ausgestaltung noch einen distalen Montageträger 40 umfasst, kann über eine Montageeinrichtung 41 ein Steckbecken 12 vorzugsweise lösbar am Montagearm 27 bzw. am Montageträger 40 befestigt sein. Das Steckbecken 12 ist über einen Zulauf 19 und einen daran angeschlossenen Zulaufschlauch 42 an eine Spüleinrichtung 20 angeschlossen. Die Spüleinrichtung 20 kann einen Behälter mit Spülfluid umfassen. Alternativ kann sie über eine Versorgungsleitung 43 mit einer Wasserzufuhr verbunden sein. Es kann auch vorgesehen sein, ein deutlich über Zimmertemperatur aufgeheiztes Spülfluid zur Anwendung zu bringen, um die Reinigung zu begünstigen.

Das Steckbecken 12 weist weiterhin einen Ablauf 17 auf, der über einen Ablaufschlauch 51 mit einer Absaugeinrichtung 18 verbunden ist. Zwischen Ablauf 17 und Absaugeinrichtung 18 ist zweckmäßigerweise noch eine nicht gezeigte Geruchssperre in Form einer Rückschlagklappe, eines U-förmigen Rohres, eines Schiebers o.ä. ausgebildet

In der Absaugeinrichtung 18 kann weiterhin eine Analyseeinrichtung 30 untergebracht sein, um menschliche Exkremente, insbesondere Stuhl bzw. Urin in herkömmlicher Weise zu analysieren, wie dies aus medizinischer Sicht bei kranken oder pflegebedürftigen Patienten oftmals angezeigt erscheint. Die Analyseeinrichtung 30 kann bedarfsweise in den Absaugstrom zwischengeschaltet sein. Alternativ kann auch eine entsprechende Probe abgezweigt und in der Analyseeinrichtung 30 untersucht werden. Selbstverständlich ist es auch möglich, nur eine Probenabzweigeinrichtung vorzusehen, um die Analyse in einem externen Labor vorzunehmen.

Um einen an das Bett 11 gebundenen Patienten die Benutzung des Steckbeckens 12 in noch bequemerer Position zu gestatten, kann die Vorrichtung weiterhin eine Luftbefüllungseinrichtung 29 umfassen, die über einen Luftbefüllungsschlauch 44 mit einer im Bett 11 positionierbaren Luftkammer 28 zusammenwirkt.

Zur Steuerung der Vorrichtung kann schließlich noch eine Steuereinrichtung 45 vorgesehen sein, die ein externes über ein Kabel 46 verbundenes Bedienteil 47 umfasst. Über das Bedienteil 47 kann der Patient oder das Pflege- bzw. Klinikpersonal Steuerbefehle an die Steuereinrichtung 45 geben, welche dann einen (nicht gezeigten) Antrieb für den Montagearm 27, die Spüleinrichtung 20, die Absaugeinrichtung 18, die Luftbefüllungseinrichtung 29 oder die Analyseeinrichtung 30 oder eine andere an der Vorrichtung bzw. im Steckbecken 12 vorgesehene Komponente ansteuert.

Der nicht dargestellte, mindestens eine Antrieb des Montagearms kann in Form eines mechanischen, pneumatischen oder hydraulischen Antriebs ausgebildet sein.

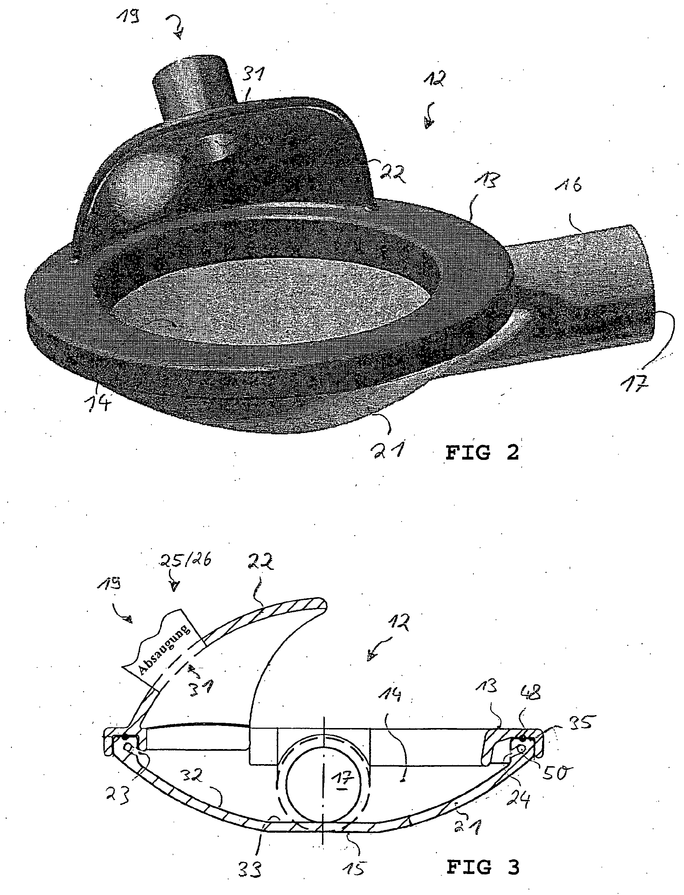

Nachfolgend wird eine erste Ausführungsform des erfindungsgemäßen Steckbeckens anhand der perspektivischen Ansicht nach FIG 2 veranschaulicht. Das Steckbecken 12 umfasst zunächst einen von einer Schüssel 21 gebildeten Aufnahmeraum 14. Am Aufnahmeraum 14 der Schüssel 21 ist der bereits erwähnte Ablauf 17 ausgebildet, der bei der vorliegenden Ausführungsform mit seiner Außenwandung auch eine Montagestelle 16 zur Montage an einem Montagearm 27 definiert.

Auf der Schüssel 21 ist eine bei der vorliegenden Ausführungsform als separates Bauteil ausgebildete, hier im Querschnitt im Wesentlichen U-förmige, umlaufende Brille 13 ausgebildet.

Die umlaufende Brille 13 kann an der Schüssel 21 dauerhaft befestigt sein. Bevorzugtermaßen ist die umlaufende Brille 13, was Ersatz und Reinigung begünstigt, lösbar ausgebildet. Besonders bevorzugt wird eine lösbare Verrastung zwischen umlaufender Brille 13 und Schüssel 21 nach Art einer Snap-in-Verbindung. Auf einer Seite des Steckbeckens 12 weist die umlaufende Brille 13 eine über die Brille 13 nach oben vorstehende hochgezogene Schutzwand 22 auf, die eine zum Aufnahmeraum 14 des Steckbeckens 12 gewölbte Krümmung aufweist und mit einer Öffnung 31 versehen ist, die mit dem bereits erwähnten Zulauf 19 des Steckbeckens in Fluidverbindung steht.

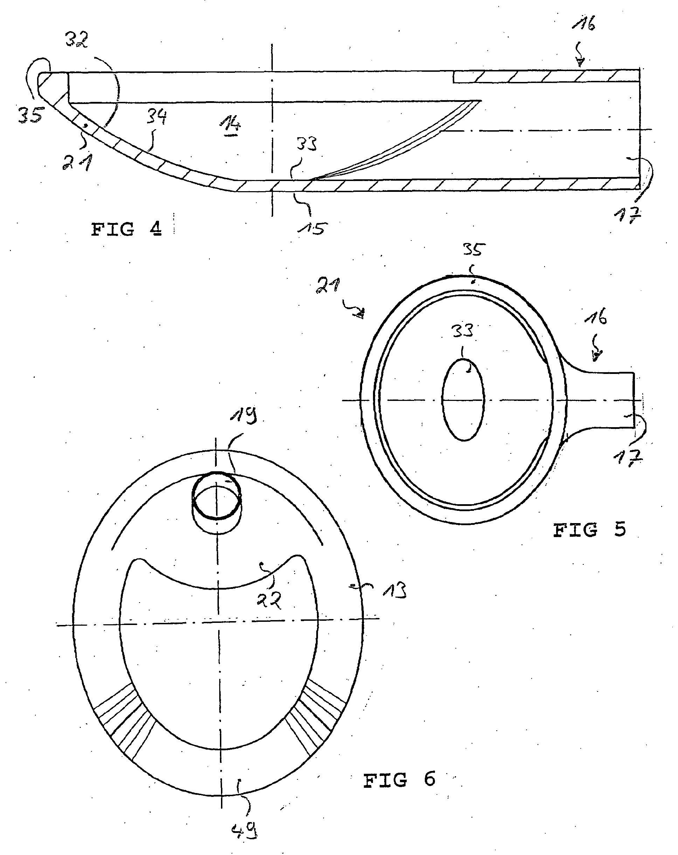

In FIG 3 ist das Steckbecken nach FIG 2 in einer ersten Schnittansicht dargestellt. Aus dieser Darstellung lässt sich der Krümmungsverlauf der Schüssel 21 entnehmen. An ihrer Außenseite bildet die Schüssel 21 eine im Wesentlichen ebene Auflagefläche 15 aus, die aber auch zur Abstützung auf einem Montagearm 17 bzw. Montageträger 40 vorgesehen sein kann. Auf der Innenseite 32 bildet die Schüssel 21 eine insgesamt glatte Innenfläche 34 aus. Im zentralen Bereich des Aufnahmeraums 14 ist ein flacher Bodenabschnitt 33 vorgesehen, wobei der flache Bodenabschnitt 33 zu einem Rand 35 der Schüssel 21 hin in einen gekrümmten Bodenbereich übergeht. Es kann insbesondere vorgesehen sein, wie in FIG 3 dargestellt, dass das Maß der Krümmung ausgehend vom flachen Bodenabschnitt 33 zum Rand 35 hin ggf. in mehrfachen Schritten diskret oder auch kontinuierlich zunimmt.

Zwischen umlaufender Brille 13 und Schüssel 21 kann eine umlaufende Dichtung 48 vorgesehen sein, um den Bereich unterhalb der umlaufenden Brille 13 gegen die Schüssel 21 abzudichten.

Nach einem weiteren bevorzugten Aspekt der vorliegenden Erfindung gehen flacher Bodenabschnitt 33 und Ablauf 17 niveaugleich ineinander über, so dass sich ein besonders günstiges Entleerungsverhalten bei Absaugung von in der Schüssel befindlichen Exkrementen ergibt.

Die bereits erwähnte Schutzwand 22 ist bei der Ausführungsform nach FIG 3 mit der umlaufenden Brille 13 einteilig ausgebildet. Die bereits erwähnte Öffnung 31 kann nicht nur der Zuführung von Spülfluid dienen, sondern auch einen Absauganschluss 25 definieren zur Geruchsabsaugung. Die Öffnung 31 kann auch diese beiden Funktionen zeitlich versetzt übernehmen sowie ggf. weitere Funktionen wie Zugabe von Geruchsbekämpfungsmitteln, Beherbergung von mechanischen Reinigungsgeräten oder Ausbildung eines Trocknungsanschlusses 26 dergestalt, dass über die Öffnung 31 Druckluft, vorzugsweise auch warme Druckluft eingeblasen wird, um die Innenfläche 34 der Schüssel 21 rasch zu trocknen. Es kann weiterhin an dem Steckbecken, insbesondere in der Öffnung 31 noch eine Patientenduscheinrichtung (nicht gezeigt) vorgesehen sein, zum Duschen/Säubern von Körperpartien. Warme Druckluft kann auch dazu vorgesehen und bereitgestellt werden, um Körperpartien beispielsweise nach einem Abduschen zu trocknen.

Aus FIG 3 geht weiterhin schematisch noch hervor, dass ein Spülfluid nicht ausschließlich über die Öffnung 31 in der Schutzwand 22 eingebracht werden muss; ergänzend oder auch alternativ können düsenartige Auslässe 23, 24 beispielsweise im Bereich unterhalb der umlaufenden Brille 13 vorgesehen sein, die durch einen umlaufenden Versorgungskanal 50 miteinander verbunden sein können. Die düsenartigen Auslässe 23, 24 bzw. der umlaufende Versorgungskanal 50 steht über eine nicht gezeigte Fluidführung mit einem Zulauf, insbesondere dem Zulauf 19 in Fluidverbindung.

In FIG 4 ist eine Schnittansicht senkrecht zur Schnittansicht nach FIG 3 dargestellt. Aus dieser Ansicht wird der niveaugleiche Übergang vom flachen Bodenabschnitt 33 zum Ablauf 17 noch besser verständlich.

In FIG 5 ist eine Draufsicht auf die Schüssel 21 nach den FIG 3 und 4 veranschaulicht, wobei hier die langgestreckte Form in einer Richtung senkrecht zur Achse des Ablaufes 17 erkennbar wird. Die Schüssel 21 weist eine elliptische Grundform auf, wobei das Verhältnis der längsten Ausdehnung zur kürzesten Ausdehnung 1,2 beträgt.

In FIG 6 ist eine abgewandelte Ausführungsform der umlaufenden Brille 13 mit daran angeschlossener Schutzwand 22 in einer Darstellung von oben veranschaulicht. An der der Schutzwand 22 gegenüberliegenden Seite der umlaufenden Brille 13 bildet die umlaufende Brille 13 eine Vertiefung 49 aus. Die so ausgebildete Vertiefung 49 erleichtert einem bettlägerigen Patienten die Benutzung des Steckbeckens 12. Die Vertiefung 49 kann auch noch mit einer Polsterung versehen sein, um die Benutzung des Steckbeckens noch weiter zu erleichtern.

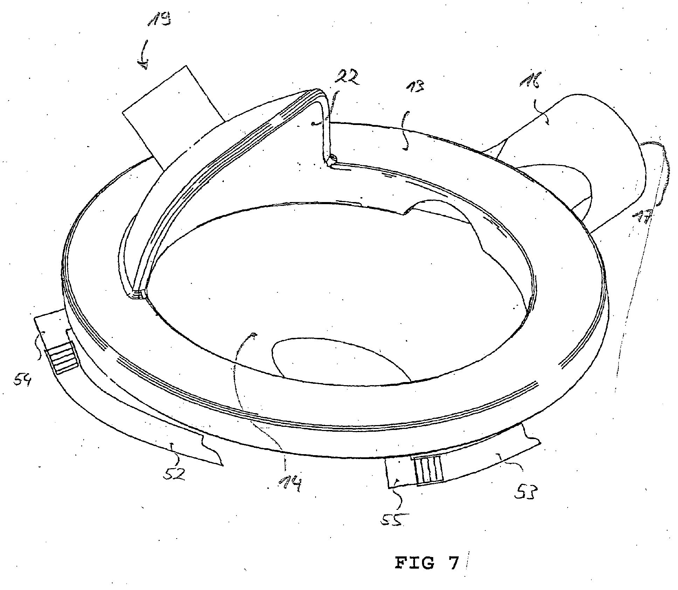

In FIG 7 ist eine modifizierte Ausführungsform des Steckbeckens nach der Erfindung veranschaulicht. Zur Versorgung des in Fig. 3 veranschaulichten Versorgungskanals 50 mit Spülfluid sind an der Außenseite der Schüssel 21 Anschlussstutzen 54, 55 vorgesehen, die Spülanschlüsse 52, 53 definieren.

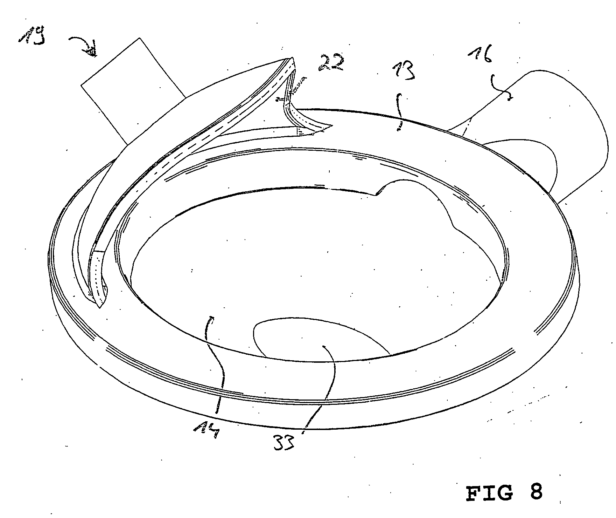

In FIG 8 ist eine nochmals modifizierte Ausführungsform der vorliegenden Erfindung veranschaulicht, bei der die Schutzwand auf der Brille 13 angeordnet ist und insbesondere als separates Bauteil auf der Brille 13 befestigt, insbesondere angeklebt sein kann.

**Bezugszeichenliste**

| | |
|---|---|
| 11 | Bett |
| 12 | Steckbecken |
| 13 | umlaufende Brille |
| 14 | Aufnahmeraum |
| 15 | Auflagefläche |
| 16 | Montagestelle |
| 17 | Ablauf |
| 18 | Absaugeinrichtung |
| 19 | Zulauf |
| 20 | Spüleinrichtung |
| 21 | Schüssel |
| 22 | Schutzwand |
| 23, 24 | düsenartige Auslässe |
| 25 | Absauganschluss |
| 26 | Trocknungsanschluss |
| 27 | Montagearm |
| 28 | Luftkammer |
| 29 | Luftbefüllungseinrichtung |
| 30 | Analyseeinrichtung |
| 31 | Öffnung (in Schutzwand) |
| 32 | Innenseite |
| 33 | flacher Bodenabschnitt |
| 34 | Innenfläche |
| 35 | Rand |
| | |
| 38 | rahmenartiges Gestell |
| 39 | Höhenverstelleinrichtung |
| 40 | Montageträger |
| 41 | Montageeinrichtung |
| 42 | Zulaufschlauch |
| 43 | Versorgungsleitung |
| 44 | Luftbefüllungsschlauch |
| 45 | Steuereinrichtung |
| 46 | Kabel |
| 47 | Bedienteil |
| 48 | umlaufende Dichtung |
| 49 | Vertiefung |
| 50 | umlaufender Versorgungskanal |
| 51 | Ablaufschlauch |
| 52, 53 | Spülanschlüsse |
| 54, 55 | Anschlussstutzen |

**Patentansprüche**

1. Zur Aufnahme menschlicher Exkremente ausgebildetes, relativ zum Bett eines pflegebedürftigen Patienten positionierbares Steckbecken mit einem durch eine umlaufende Brille (13) teilweise abgedeckten Aufnahmeraum (14), umfassend:

- eine Montagestelle (16) zur lösbaren Befestigung des Steckbeckens (12) an einem vorzugsweise verfahrbaren Montagearm (27),
- einen Ablauf (17) zum Anschluss an eine Absaugeinrichtung (18) und
- einen Zulauf (19) zum Anschluss an eine Spüleinrichtung (20).

2. Steckbecken nach Anspruch 1,
   **dadurch gekennzeichnet,**
   **dass** Zulauf (19) und Ablauf (17) um 20° bis 160°, vorzugsweise etwa 70° bis 110°, insbesondere etwa 90° zueinander versetzt angeordnet sind.

3. Steckbecken nach Anspruch 1 oder 2,
   **dadurch gekennzeichnet,**
   **dass** die Schüssel (21) bzw. die Brille (13) an einer Seite zu einer Schutzwand (22) hochgezogen ist.

4. Steckbecken nach einem der Ansprüche 1 bis 3,
   **dadurch gekennzeichnet,**
   **dass** die umlaufende Brille (13) lösbar auf der Schüssel (21) befestigt ist.

5. Steckbecken nach einem der Ansprüche 1 bis 4,
   **dadurch gekennzeichnet,**
   **dass** der Zulauf (19) mit einer Öffnung (31) in der Schutzwand (22) in Fluidverbindung steht.

6. Steckbecken nach einem der Ansprüche 1 bis 5,
   **dadurch gekennzeichnet,**
   **dass** der Zulauf (19) mit einer, vorzugsweise einer Mehrzahl düsenartiger Auslässe (23, 24) in Fluidverbindung steht.

7. Steckbecken nach einem der Ansprüche 1 bis 6,
   **dadurch gekennzeichnet,**
   **dass** in der Schüssel (21) und/oder im Bereich der Schutzwand (22) ein Absauganschluss (25) für einen Geruchsabzug vorgesehen ist.

8. Steckbecken nach einem der Ansprüche 1 bis 7,
   **dadurch gekennzeichnet,**
   **dass** in der Schüssel (21) oder im Bereich der Schutzwand (22) ein Trocknungsanschluss (26) vorgesehen ist, um vorzugsweise durch einen Luftstrom eine Trocknung des Steckbeckens nach Gebrauch zu bewirken.

9. Steckbecken nach einem der Ansprüche 1 bis 8,
   **dadurch gekennzeichnet,**
   **dass** die Schüssel (21) in ihrer Projektion in Gebrauchslage von oben eine ovale oder elliptische Grundform aufweist mit maximaler Ausdehnung in eine erste Vorzugsrichtung und einer minimalen Ausdehnung in eine zweite Vorzugsrichtung, wobei erste und zweite Vorzugsrichtung zueinander orthogonal liegen.

10. Steckbecken nach einem der Ansprüche 1 bis 9,
    **dadurch gekennzeichnet,**
    **dass** der Ablauf (17) an einer Schmalseite der Schüssel (21) angeordnet ist.

11. Steckbecken nach einem der Ansprüche 1 bis 10,
    **dadurch gekennzeichnet,**
    **dass** die Innenseite (32) der Schüssel (21) einen im Wesentlichen flachen Bodenabschnitt (33) umfasst.

12. Steckbecken nach Anspruch 11,
    **dadurch gekennzeichnet,**
    **dass** die Krümmung der Innenfläche (34) der Schüssel (21) von dem im Wesentlichen flachen Bodenabschnitt (33) nach oben hin zu einem Rand (35) der Schüssel (21) zunimmt.

13. Steckbecken nach einem der Ansprüche 1 bis 12,
    **dadurch gekennzeichnet,**
    **dass** an der Schüssel (21) mechanische Reinigungsgeräte angeordnet sind.

14. Steckbecken nach einem der Ansprüche 1 bis 13,
    **dadurch gekennzeichnet, dass**
    an der Unterseite des Steckbeckens eine Auflagefläche (15) zur Auflage des Steckbeckens (12) auf einem Bett (11) des Patienten vorgesehen ist.

15. Vorrichtung zur Absaugung von Exkrementen eines an ein Bett (11) gebundenen Patienten, umfassend ein Steckbecken nach einem der Ansprüche 1 bis 14,
    einen Montagearm (27), an dem das Steckbecken (12) befestigt ist und relativ zu einem Bett (11) positionierbar ist,
    eine Absaugeinrichtung (18), die am Ablauf (17) des Steckbeckens (12) angeschlossen ist und
    eine Spüleinrichtung (20), die mit dem Zulauf (19) des Steckbeckens in Fluidverbindung steht.

16. Vorrichtung nach Anspruch 15,
    **dadurch gekennzeichnet,**
    **dass** zwischen der Absaugeinrichtung (18) und dem Steckbecken (12) eine Geruchssperre angeordnet ist.

17. Vorrichtung nach Anspruch 15 oder 16,
    **dadurch gekennzeichnet,**
    **dass** der Montagearm (27) schwenkbar und/oder längenverstellbar, vorzugsweise automatisch einstellbar, ausgebildet ist.

**18.** Vorrichtung nach einem der Ansprüche 15 bis 17, **dadurch gekennzeichnet,** **dass** weiterhin eine auf dem Bett (11) unterhalb des Patienten positionierbare Luftkammer (28) vorgesehen ist und mit einer Luftbefüllungseinrichtung (2), wie einem Kompressor, in Wirkverbindung steht derart, dass der Patient im Bereich seines Rücken bzw. der Lendenwirbelsäule angehoben werden kann.

**19.** Vorrichtung nach einem der Ansprüche 15 bis 18, **dadurch gekennzeichnet,** **dass** in Strömungsrichtung vor oder nach der Absaugeinrichtung (18) eine Analyseeinrichtung (37) zur Analyse von bestimmten Parametern der ausgeschiedenen Exkremente, wie pH-Bestimmung des Urins etc. zwischengeschaltet bzw. zwischenschaltbar ist.

FIG 1

19 2

31

22 12 13 16

17

14

21 FIG 2

25/26 22

19

Absaugung

12

31

14 13 48

35

23

32

17

50

24

33 15 21

FIG 3

FIG 4

FIG 5

FIG 6

FIG 7

FIG 8

**Europäisches Patentamt**

# EUROPÄISCHER RECHERCHENBERICHT

Nummer der Anmeldung

EP 02 02 6250

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X<br>Y | GB 668 213 A (ISADORE LEVIN)<br>12. März 1952 (1952-03-12)<br>* Seite 1, Zeile 48 - Zeile 72 *<br><br>* Seite 2, Zeile 87 - Zeile 105 *<br>* Seite 2, Zeile 128 - Seite 3, Zeile 9 *<br>* Seite 3, Zeile 27 - Zeile 39 *<br>* Seite 3, Zeile 83 - Zeile 116;<br>Abbildungen *<br>--- | 1,2,4,6,<br>9-14<br>3,5,7,8,<br>15,16 | A61G9/00<br>A61B10/00<br>B01L3/00 |
| Y | US 5 647 090 A (YANG SUNG HWA)<br>15. Juli 1997 (1997-07-15)<br>* Spalte 5, Zeile 24 - Zeile 36 *<br>* Spalte 7, Zeile 60 - Spalte 8, Zeile 7;<br>Abbildungen *<br>--- | 3,5,7,8,<br>15,16 | |
| A | US 2 614 267 A (PERRI FRANK B)<br>21. Oktober 1952 (1952-10-21)<br>* Spalte 2, Zeile 44 - Spalte 3, Zeile 4 *<br>* Spalte 4, Zeile 32 - Zeile 46;<br>Abbildungen *<br>--- | 17 | |
| A | EP 0 494 488 A (KAWASAKI SEIJI)<br>15. Juli 1992 (1992-07-15)<br>* Spalte 9, Zeile 22 - Zeile 35; Abbildung<br>7 *<br>--- | 18 | RECHERCHIERTE SACHGEBIETE (Int.Cl.7)<br><br>A61G<br>E03D<br>A61B<br>B01L |
| A | US 5 423 792 A (OXLEY L THOMAS)<br>13. Juni 1995 (1995-06-13)<br>* Spalte 7, Zeile 51 - Zeile 63; Ansprüche<br>6,11; Abbildungen *<br>--- | 19 | |
| A | US 4 065 179 A (TAKASAKI TAKAO)<br>27. Dezember 1977 (1977-12-27)<br>* Spalte 2, Zeile 45 - Zeile 54 *<br>* Spalte 4, Zeile 24 - Zeile 31;<br>Abbildungen *<br>---<br>-/-- | 1,15,17 | |

Der vorliegende Recherchenbericht wurde für alle Patentansprüche erstellt

| Recherchenort | Abschlußdatum der Recherche | Prüfer |
|---|---|---|
| DEN HAAG | 25. April 2003 | Cametz, C |

**Europäisches Patentamt**

## EUROPÄISCHER RECHERCHENBERICHT

Nummer der Anmeldung

EP 02 02 6250

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| A | WO 88 03789 A (EGGERT HEINZ) 2. Juni 1988 (1988-06-02) * Seite 3, Zeile 16 - Seite 4, Zeile 5; Abbildungen * --- | 1,15,17 | |
| A | WO 95 04513 A (FUNATUKI CHIFUYU ;KANAI SUMIYO (JP)) 16. Februar 1995 (1995-02-16) * Zusammenfassung * ----- | | |

**RECHERCHIERTE SACHGEBIETE (Int.Cl.7)**

Der vorliegende Recherchenbericht wurde für alle Patentansprüche erstellt

| Recherchenort | Abschlußdatum der Recherche | Prüfer |
|---|---|---|
| DEN HAAG | 25. April 2003 | Cametz, C |

EPO FORM 1503 03.82 (P04C03)

**Europäisches**

**Patentamt**

Nummer der Anmeldung

EP 02 02 6250

## GEBÜHRENPFLICHTIGE PATENTANSPRÜCHE

Die vorliegende europäische Patentanmeldung enthielt bei ihrer Einreichung mehr als zehn Patentansprüche.

☐ Nur ein Teil der Anspruchsgebühren wurde innerhalb der vorgeschriebenen Frist entrichtet. Der vorliegende europäische Recherchenbericht wurde für die ersten zehn sowie für jene Patentansprüche erstellt, für die Anspruchsgebühren entrichtet wurden, nämlich Patentansprüche:

☐ Keine der Anspruchsgebühren wurde innerhalb der vorgeschriebenen Frist entrichtet. Der vorliegende europäische Recherchenbericht wurde für die ersten zehn Patentansprüche erstellt.

## MANGELNDE EINHEITLICHKEIT DER ERFINDUNG

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

Siehe Ergänzungsblatt B

☐ Alle weiteren Recherchengebühren wurden innerhalb der gesetzten Frist entrichtet. Der vorliegende europäische Recherchenbericht wurde für alle Patentansprüche erstellt.

☒ Da für alle recherchierbaren Ansprüche die Recherche ohne einen Arbeitsaufwand durchgeführt werden konnte, der eine zusätzliche Recherchengebühr gerechtfertigt hätte, hat die Recherchenabteilung nicht zur Zahlung einer solchen Gebühr aufgefordert.

☐ Nur ein Teil der weiteren Recherchengebühren wurde innerhalb der gesetzten Frist entrichtet. Der vorliegende europäische Recherchenbericht wurde für die Teile der Anmeldung erstellt, die sich auf Erfindungen beziehen, für die Recherchengebühren entrichtet worden sind, nämlich Patentansprüche:

☐ Keine der weiteren Recherchengebühren wurde innerhalb der gesetzten Frist entrichtet. Der vorliegende europäische Recherchenbericht wurde für die Teile der Anmeldung erstellt, die sich auf die zuerst in den Patentansprüchen erwähnte Erfindung beziehen, nämlich Patentansprüche:

**Europäisches**
**Patentamt**

**MANGELNDE EINHEITLICHKEIT**
**DER ERFINDUNG**
**ERGÄNZUNGSBLATT B**

Nummer der Anmeldung

EP 02 02 6250

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

1. Ansprüche: 1-14

   Steckbecken mit einem durch eine umlaufende Brille teilweise abgedeckten Aufnahmeraum, umfassend eine Montagestelle, einen Ablauf, einen Zulauf

2. Ansprüche: 15-19

   Vorrichtung mit einem Montagearm, an dem ein Steckbecken befestigt ist, und mit einer Absaugeinrichtung, die am Ablauf des Steckbeckens angeschlossen ist.

**ANHANG ZUM EUROPÄISCHEN RECHERCHENBERICHT**
**ÜBER DIE EUROPÄISCHE PATENTANMELDUNG NR.**            EP 02 02 6250

In diesem Anhang sind die Mitglieder der Patentfamilien der im obengenannten europäischen Recherchenbericht angeführten
Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

25-04-2003

| Im Recherchenbericht angeführtes Patentdokument | | | Datum der Veröffentlichung | Mitglied(er) der Patentfamilie | | | Datum der Veröffentlichung |
|---|---|---|---|---|---|---|---|
| GB 668213 | A | | 12-03-1952 | KEINE | | | |
| US 5647090 | A | | 15-07-1997 | KEINE | | | |
| US 2614267 | A | | 21-10-1952 | KEINE | | | |
| EP 0494488 | A | | 15-07-1992 | EP | 0494488 | A1 | 15-07-1992 |
| US 5423792 | A | | 13-06-1995 | US | 5084041 | A | 28-01-1992 |
| | | | | US | 5160329 | A | 03-11-1992 |
| | | | | US | 5284772 | A | 08-02-1994 |
| | | | | AU | 7779591 | A | 11-11-1991 |
| | | | | WO | 9116086 | A1 | 31-10-1991 |
| | | | | AU | 8423991 | A | 04-02-1992 |
| | | | | WO | 9200703 | A1 | 23-01-1992 |
| | | | | US | 5217443 | A | 08-06-1993 |
| US 4065179 | A | | 27-12-1977 | KEINE | | | |
| WO 8803789 | A | | 02-06-1988 | AT | 387512 | B | 10-02-1989 |
| | | | | AT | 314286 | A | 15-07-1988 |
| | | | | WO | 8803789 | A1 | 02-06-1988 |
| | | | | AT | 75597 | T | 15-05-1992 |
| | | | | DE | 3778886 | D1 | 11-06-1992 |
| | | | | EP | 0296185 | A1 | 28-12-1988 |
| WO 9504513 | A | | 16-02-1995 | JP | 7051326 | A | 28-02-1995 |
| | | | | JP | 7051327 | A | 28-02-1995 |
| | | | | JP | 7059819 | A | 07-03-1995 |
| | | | | JP | 7017223 | U | 28-03-1995 |
| | | | | JP | 7031032 | U | 13-06-1995 |
| | | | | AU | 7350394 | A | 28-02-1995 |
| | | | | WO | 9504513 | A1 | 16-02-1995 |

EPO FORM P0461

Für nähere Einzelheiten zu diesem Anhang : siehe Amtsblatt des Europäischen Patentamts, Nr.12/82